(19)

Europäisches
Patentamt
European
Patent Office
Office européen
des brevets

(11) **EP 2 187 327 A1**

(12) **EUROPEAN PATENT APPLICATION**

(43) Date of publication:
**19.05.2010 Bulletin 2010/20**

(51) Int Cl.:
*G06F 19/00* (2006.01)       *G06F 3/048* (2006.01)

(21) Application number: **08105816.6**

(22) Date of filing: **18.11.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicants:
• **F. Hoffmann-La Roche AG**
**4070 Basel (CH)**
Designated Contracting States:
**AT BE BG CH CY CZ DK EE ES FI FR GB GR HR
HU IE IS IT LI LT LU LV MC MT NL NO PL PT RO
SE SI SK TR**
• **Roche Diagnostics GmbH**
**68305 Mannheim (DE)**
Designated Contracting States:
**DE**

(72) Inventor: **Avnur, Arie**
**4052 Basel (CH)**

(74) Representative: **Poredda, Andreas**
**Roche Diagnostics AG**
**Forrenstrasse**
**6343 Rotkreuz (CH)**

(54) **Method for graphically processing displayed data records for minimizing a selection error rate**

(57)     The invention refers to a method for displaying and processing clinical data. In order to reduce the error rate in selecting a data record from a selection list displayed comprising similar data records it is proposed that the displayed data records that are displayed in the displayed selection list are automatically checked for similar displayed data records' representation, wherein a similar displayed representation comprises at least one displayed data record attribute that is identical or similar to the displayed data record attribute of said data type of another displayed data record, and wherein, e.g. either statically OR when a cursor is set by the user to a displayed data record representation pointed by the cursor for preparing selection of the pointed data record representation from the displayed selection list, the display of the pointed data record and/or of the similar displayed data records is additionally graphically processed upon displaying the selection list providing visual differentiation of similar record representations for the selection about to be made. Furthermore, the invention specifies a method for calculating and recording selection confidence level and records similar to the selected one for downstream verification in case further processing detects conflicts created by erroneous selection.

| Patient ID | Last Name | First Name | Age | Gender |
|---|---|---|---|---|
| 1234567 | David | Larry | 66 | M |
| 1936022 | Doe | John | 21 | M |
| 1936827 | Doe | Jeff | 54 | M |
| 3958133 | Eckerman | John | 34 | M |

**Fig. 5**

EP 2 187 327 A1

**Description**

This invention relates to a method for displaying and processing data. The method may for example be applied in displaying and processing clinical data to be selected in the use of a laboratory instrument for performing an analysis of a sample with respect to a medical significant parameter, in particular a method for displaying and processing patient medical information.

Such a laboratory instrument and a laboratory information system is usually used in a clinical or research laboratory for analyzing large amounts of samples, e.g. in a laboratory system of an analytical chemistry processing center. The instrument may be useful in the performance of a single or numerous chemical, biochemical and biological assays and reactions.

Modern research and clinical laboratory procedures include biological and chemical analysis of specimen-substances that require extensive fluid manipulations. Many of the routine applications used for analysis are bioassays, immunoassays, viral assays, mitogen assays, serology, protein assays, lymphokine assays, and sample aliquoting. Experimental biological and clinical research includes the use of photometric analysis of chemical reactions after the reactions have reached equilibrium or a fixed end point. Certain enzyme assays require a two-point or multi-point analysis embodying kinetic assays.

An important field is analyzing samples with a nucleic acid amplification technique. The purpose of the analysis is the detection (presence or absence of an analyte) and/or the quantification of the concentration of an analyte in samples. The analyte may be a nucleic acid: RNA or DNA or derivatives there off. The derivatives (Nucleic Acids, NA) mentioned include molecules which are accessible directly or indirectly (e.g. after chemical modification) to a NA amplification method (e.g. DNA-polymerase, Transcriptase, Reverse-Transcriptase, etc.). The target analyte can be e.g. genetic material with biological origin e.g. for genetic testing, in case of infectious diseases the analyte can be nucleic acid material from a virus or bacteria, in case of gene-expression the analyte can be m-RNAs, the analyte can also be methylated DNA.

The analysis is performed by a laboratory instrument for processing samples, e.g. by conducting thermocycled amplification of polynucleotides, including polymerase chain reaction.

Such a laboratory instrument is usually part of a laboratory system comprising a laboratory information system which is connected to a plurality of laboratory instruments. The laboratory instrument and optionally the laboratory system for performing such laboratory procedures require management of data related to the use of the laboratory instrument and the analysis result obtained therewith, e.g. management of sample data, process liquids and reagents used in the analysis, analysis data and patient data. These data are usually stored in a repository data base of the laboratory system. Although these data are managed to an extent as far as possible in order to save costs and to increase the reliability of the process, it is in many cases required that a user selects for further processing a data record of several data records which are displayed to him, e.g. for selecting a specific patient or a specific analysis process he wants to have performed by the instrument.

In standard methods for displaying and processing data to be selected in the use of a laboratory instrument for performing an analysis of a sample with respect to a medical significant parameter, in particular a method for displaying and processing patient medical information, the data are stored in a repository database of the instrument or of the laboratory system, which database may be located at an instrument itself and/or a database located externally on a server which is accessed by a network. The data are searched or filtered according to a query to the repository database in order to retrieve a number of data records from the data stored in the repository database. Such a data record comprises a number of data record attributes, wherein a data record attribute is an instance of a data type stored in the repository database, wherein the data type is given by data type qualifiers. An instance of a data type is for example a specific data of a specific information data type stored in the repository database, wherein the information data type is given by data type qualifiers that are representative of the specific information data types recorded in the repository database. E.g. an attribute of a patient or person record could be their first or given name, their last or family name, etc. A selection list comprising at least some of the preselected data records is displayed to a user for enabling the user to select one of the displayed data records, wherein the displayed selection list comprises a record representation for each of the selection record candidates. That record representation is typically constructed from one or more of the record attributes; e.g. first and last name (most common), ID number, etc. The data record representation can also be constructed partly or overall from derivations of data record representations. Derivations of data record representations are attribute values calculated from record attributes, like when age is calculated from date of birth, postal code from town, last name length truncated, etc.. In particular, the displayed selection list may comprise at least some of the data record attributes which are available overall.

However, in the process of selecting a data record from a displayed selection list comprising several data records the user may undertake a selection error resulting in the selection of an erroneous data record instead of the correct one. In many cases, users of data management systems select data records from a repository data base. Typically, this is done by selecting a data record from a displayed selection list. The displayed list of "candidates" of which one has to be selected comprises several data records retrieved from the database as a result of a request to the database by a search of filtering for providing the display. In some embodiments the search may be of the type "display all the data records".

In many cases similar data records appear next to each other in the selection list, especially since most selection lists are sorted by name or a similar attribute (data type qualifier). It is then not inconceivable that the user might select the wrong data record, e.g. by pointing it with a cursor.

Prior art systems do not provide means to prevent errors of this type. A handling of patient data is disclosed in US 6,775,670 and US 7,165,221. However, these references are not related to the preventing of errors in selecting data records from a displayed selection list comprising data records. At the most, when a risky selection is made which is identified in that the chosen selection would cause a risky action, a prior art system provides a confirmation form to the user. In contemporary systems the user get used to fixed pattern interactions of the interface and will confirm their selection "automatically" often, rendering this known measure of error rate reduction not very effective. Beyond this simple measure, current systems do not provide any measure to prevent selection errors. This situation is exastrebated by the time stress under which data is often entered and selected in actual clinical lab operation and many similar situations. Accordingly there is a noticeable error rate in data record selection, especially when data records with similar representation or data record attributes are displayed in the selection list. Reduction of this error rate at an acceptable cost will result in benefits in reduced health care errors and cost and all the way of saving lives.

It is therefore an object of the present invention to provide a method and a user interface for preventing errors in selecting data records from a displayed selection list comprising data record representations and or at least to reduce the error rates involved in such a selection process.

A method meeting these needs is provided according to the invention by a method according to claim 1. In such a method for displaying and processing data, e.g. clinical data to be selected in the use of a laboratory instrument for performing an analysis of a sample with respect to a medical significant parameter, in particular a method for displaying and processing general or clinical information, wherein data are stored in a repository database, the data are searched or filtered according to a query to the repository database in order to retrieve a number of data records from the data stored in the repository database, wherein a data record representation comprises a number of data record's attributes, and a data record attribute is an instance of a data type stored in the repository database as part of or linked to the data record. A data record attribute is a data element of a record; e.g. first name, last name, age, gender, etc.. The data type may be given by data type qualifiers that are representative of the data types recorded in the repository database. A selection list comprising at least some of the retrieved data records' attributes as their representation is displayed to a user for enabling the user to select one of the data records. The displayed selection list comprises record representations, each data record representation comprises at least some of the data attributes which are stored in the repository database. Data record representations which are displayed in the displayed selection list are automatically checked for similarity, wherein a similar data record representation comprises at least one data record attribute of a data type that is identical or similar to a data record attribute of said data type of another data record or creates a similar appearance that makes them difficult to distinguish, by evaluating the text distance or preferably weighted distance between representations.

The text distance is the number of characters that are different between two strings, compared for each location of the strings.

The weighted text distance is a string distance, where a difference between two characters is weighted by the visual similarity of the characters. E.g. the distance between the visually similar letters "I" and "l" will be counted as half a point, while the distance between the visually distinct letters "I" and "X" will be counted as one point. This method produces a better assessment of strings distance or similarity for the purpose of this invention.

The display of similar displayed data records is additionally graphically processed upon displaying the selection list. Graphically processing can e.g. be achieved by changing the graphical attribute of displayed data record attributes. The graphical attribute of displayed text is e.g. font, color, reversed color, underscore, and any combination thereof. The graphical processing can be initiated in any suitable manner. If for example a last name is queried all other data records having the same last name can be automatically graphically processed.

According to a preferred embodiment the display of the similar displayed data record representations is additionally graphically processed upon displaying the selection list. A cursor may be set by the user to a displayed data record pointed by the cursor for preparing selection of the pointed data record from the displayed selection list. The selection of the pointed data record may be done e.g. by clicking or double clicking a button, e.g. of the mouse used for moving the cursor, when the representation of the data record to be selected is pointed by the cursor.

Additional processing of the selection list may be done by the database server through database programmed functions, by business logic layer between the database and the client or by the client. Additional processing includes the detection of similar and potentially confusing representations in the selection list, the identification of the similar or confusing parts (similar strings). The graphical differentiation of the similar part can be determined by processing at the server side or at the client, all depending on system architecture, and all in accordance of this invention.

The invention is based on the finding that it is possible to prevent errors in selecting data records from a displayed selection list comprising data record representations and to reduce the error rates involved in such a selection process by affecting the presentation of data selection menus and providing distinctive graphical differentiation of those representation to the user.

The invention further concerns a user interface of a laboratory instrument or a laboratory system supporting displaying and processing clinical data, e.g. clinical data to be selected in the use of a laboratory instrument for performing an analysis of a sample with respect to a medical significant parameter, in particular for displaying and processing patient medical information. The user interface comprises a processor for searching or filtering data that are stored in a repository database of the instrument or system according to a query to the repository database in order to retrieve a number of data records from the data stored in the repository database. A data record comprises a number of data record attributes. A data record attribute in an instance of a data type stored in the repository database and the data type being given by data type qualifiers. The user interface further comprising a display for displaying to a user a selection list comprising at least some of the preselected data record representations for enabling the user to select one more of the displayed data records. The displayed selection list comprises at least some of the data record attributes of the data records which are displayed in the selection list. A processor automatically checks the displayed data record representations that are displayed in the displayed selection list for similar data records. A similar data record comprises at least one data record attribute that is identical or similar to the data record attribute of said data type of another data record, and the display of the data record and/or of the similar data records is additionally graphically processed.

The invention is also directed to a system comprising an analytical instrument, a terminal for displaying data to a user, a repository database and a user interface as described above.

Further the invention is directed to a computer-readable storage device containing a set of instructions that causes a computer to perform a process according to the invention.

According to a further embodiment a downstream verification can be performed. If down the road, i.e. in processing the data record selected and performing an analysis, a test result is reported that doesn't quite agree with the selected record e.g. patient's data or demographics, like his age or gender, this could point to an error in patient selection when the order was created by the selection. A couple of actions which are described in more detail in the following paragraphs can be taken to remedy this situation.

According to a preferred embodiment of the invention displayed data records that are represented in the displayed selection list are automatically checked for similar represented data records, wherein a similar represented and displayed data record comprises at least one displayed data record attribute of a data type that is identical or similar to the displayed data record attribute of said data type of another displayed data record.

According to a method according to the invention upon confirming the selection of a specific data record by the user a confidence level of the selection is recorded together with the selected data record for enabling a downstream verification, wherein a higher confidence level is assigned to the selected data record the more different the data record representations in the displayed selection list have been when the data record was selected and wherein a lower confidence level is assigned to the selected data record the more similar the data record representations in the displayed selection list have been when the data record was selected or the higher the number of similar data record representations has been when the data record was selected. The confidence level can be checked if a plausibility check is not passed upon performing the analysis with the selected data record and the user is prompted an error message when the confidence level assigned is low. If the data record was selected out of very different ones, like in normal cases, it is assigned a high confidence level. If it was selected from a few similar record representations, its confidence level is lower. Various models and formulae can be used for assessing the confidence level according to the above description. One will be:

$$SCL = 1 - 1 / (K * (RD/RT) * DH * UC)$$

where:

SCL stands for Selection Confidence Level,
K is a proportion factor,
RD is the text distance or weighted text distance between the records, (see definitions)
RT is the total representation string length
DH is the level that distance is highlighted. DH = 1 if the differentiating parts of the records' representation are displayed with the same graphics attributes as the equal parts or as the rest of the selection list. DH > 1 if the discriminating text is highlighted in a way that helps the user distinguish between the records and identify the one the user is looking for, and
UC is a user dependent factor; some users will tend to make more errors than others under the same conditions.

When a data item having a selection confidence level below a selection confidence threshold is encountered a warning may be issued for the user to reconsider the selection. The selection confidence threshold may be set by the producer of the software or may be set by an administrator.

According to a second method upon confirming the selection of a data record by the user, similar data records (e.g. similar data records which are displayed in the selection list, when a data record was selected) are recorded together with the selected data record for enabling a downstream verification or correction. The recorded similar data records can be displayed to the user and an error message can be prompted to the user if a plausibility check is not passed. A plausibility check can be made by comparing an analytical result with stored reference data for patients of e. g. same gender, age and so on. Later on, when conflicting data is encountered, it can be checked against those data records recorded together with the selected one, and alternative selection from these data records may be offered to the user for reconsideration.

Further details and advantages of the present invention are illustrated in the following based on exemplary embodiments making reference to the attached drawings. The following is depicted in the figures:

Fig. 0    Hierarchy of a typical data management system;

Fig. 1    shows a system according to the invention;

Fig. 2    shows a first selection list of data records with patient data;

Fig. 3    shows a second selection list of data records with patient data;

Fig. 4    shows a data record selection warning according to a first embodiment of the invention comprising a highlighted data record differentiation representation;

Fig. 5    shows a first selection list of data records with patient data according to the invention comprising a highlighted data record differentiation representation;

Fig. 6    shows a second selection list of data records with patient data according to the invention comprising a dynamic data record representation;

Fig. 7    shows a third selection list of data records with patient data according to the invention comprising another dynamic data record representation.

The following description of the embodiments is merely exemplary in nature and is in no way intended to limit the invention, its application, or uses. For example, the present invention may find utility in a wide variety of applications of laboratory instruments.

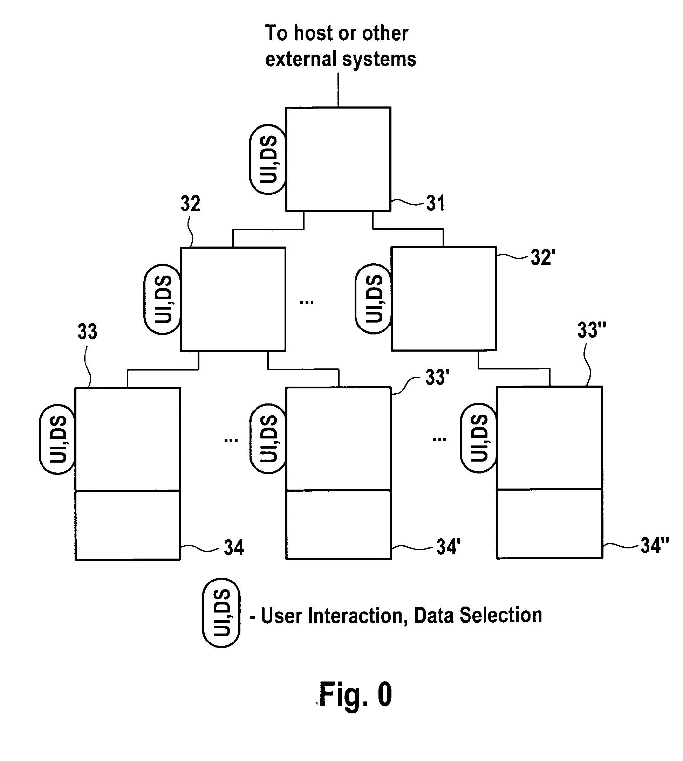

Figure 0 shows the hierarchy of a typical data management system. It comprises a top level data management system 31 e.g. a Laboratory Information System that manages data for the whole lab, and lower level, more specific data system 32, 32' for sections of the operation, e.g. a Work Area Management system. It also interfaces with lower level specific systems 33, 33', 33" that consume and produce data e.g. interfaces of laboratory analyzers, scanner, printer 34, 34', 34", through their data system. User interaction through user interface and data selection are needed at different points at each of those system at any level as indicated by UI, DS.

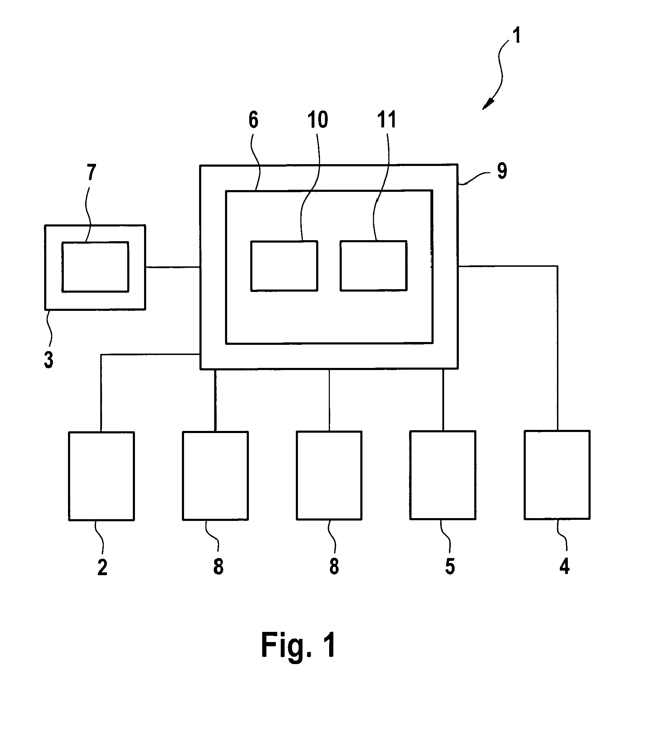

Figure 1 shows a laboratory system 1 comprising an analytical instrument 2, a terminal 3 for displaying data to a user and a repository database 4, a further optional repository database 5, and a user interface 6 according to the invention. The terminal 3 comprises a display 7. Further the laboratory system 1 may comprise several laboratory instruments 8. Such a laboratory instrument 8 is usually used in a clinical or research laboratory for analyzing large amounts of samples, e.g. in a laboratory system 1 of an analytical chemistry processing center. The laboratory instrument 8 may be useful in the performance of a single or numerous chemical, biochemical and biological assays and reactions.

The laboratory system 1 comprises a laboratory information system 9, and the terminal 3 is connected to the laboratory information system 9 and/or directly to one or several analytical instruments 8. The user interface 6 comprises a communication processor 10 and a display processor 11.

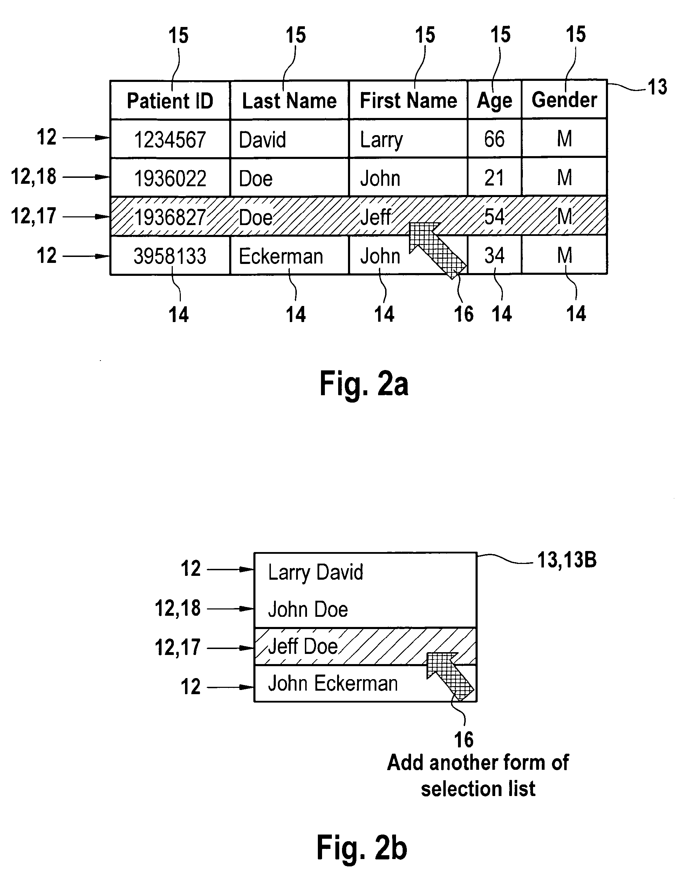

Figure 2a illustrates a table/grid embodiment for the selection of a patient data record 12 to be assigned to a specific analysis order performed or to be performed by a laboratory instrument, wherein the record of the patient from whom the sample for analysis was taken has to be selected and assigned to the order. Such a selection is a common task, and a critical one. Selecting and assigning the wrong patient to an order creates a potentially dangerous situation.

The selection list 13 displayed in figure 2a is used for displaying and processing medical data. Such data is typically selected in the use of a laboratory instrument 8 for performing an analysis of a sample with respect to a medically significant parameter or for retrieving medical data. The data are stored in a repository database 4 and the data are searched or filtered according to a query to the repository database in order to select a number of data records 12 from the data stored in the repository database.

The selection list 13 shown comprises at least some of the selected data records' representation, wherein a data record 12 in the representation of figure 2a is a line in the selection list 13 , and is displayed to a user for enabling the user to select one of the displayed data records 12. Therein the displayed selection list 13 comprises at least some of the data record attributes 14 of the data records 12, e.g. of the data records 12 which are displayed in the selection list 13. Not all data record attributes relating to a data record 12 have to be displayed in the selection list 13.

Figure 2b depicts a menu type list (like in a drop down menu) embodiment, both types, table/grid and menu type list are commonly used for record selection. The data type qualifiers 15 are patient ID, last name, first name, age and gender. Four data records 12 are displayed in the selection list 13B, namely the data records of four patients Larry David, John Doe, Jeff Doe and John Eckerman. The data record entries comprise patient or clinical data according to the data type qualifiers 15. The data record 12 of Jeff Doe is preselected by a pointer or cursor 16 placed on his data record 12 by the user of the laboratory system 1, laboratory information system 9 or analytical instrument 8. Therefore the data record 12 of Jeff Doe can be named a pointed data record 17. The preselection can also be performed in another suitable manner, e.g. by querying a last name.

Generally it may be useful if at least some of the data type qualifiers 15 of the data record attributes 14 displayed in the selection list 13 13 B are displayed together with the displayed data records 12 and the displayed data record entries 14.

Preferably, the data record attributes 14 may relate to data of patients. Further preferably the data type qualifiers 15 represent various patient record properties like patient id, patient first name, patient last name, patient age, patient gender, patient street of the address, patient city of address, patient state of address and medical information associated with a patient. Medical information may be disease history, present and past treatments, etc.

In many cases similar data records 18 appear next to each other in the selection list, especially since most selection lists 13, 13B are sorted by name or similar attributes. A similar displayed data record 18 is **characterized in that** it comprises at least one displayed data record attribute 14 of a data type that is identical or similar to the displayed data record attribute of said data type of another displayed data record. In figure 2 the data records 12 of John Doe and Jeff Doe are similar, because the data record attribute 14 "Doe" of the data type "last name" is identical. Therefore the data record 12 displayed of John Doe is a similar data record 18 with respect to the (pointed) data record 17 of Jeff Doe. The data record 12 of Larry David however is not a similar data record 18, i.e. similar to the data record 12 of Jeff Doe, although it comprises an identical data record attribute 14 of the type gender which is M, because the gender is not given much weight in evaluating whether a data record 12 is a similar data record 18 to another data record 12 or to the pointed data record 17.

It is then not inconceivable that the user might point and select the wrong data record 12, i.e. the pointed data record 17 of Jeff Doe instead the similar data record 18 of John Doe, thus neglecting or overseeing other displayed and differing data record entries 14 comprised in the displayed data records 12 that would enable him to check his selection further. In figure 2 the user might disregard in selecting the erroneous data record 12 the differing data record entries 14 of the data type "age" of Jeff Doe which is 54 and John Doe which is 21. This could happen as a result of environmental conditions (like task and time pressure), user ability to discriminate between similar lines (user specific performance), and the similarity among data records 12. Such selection errors result in high error rates.

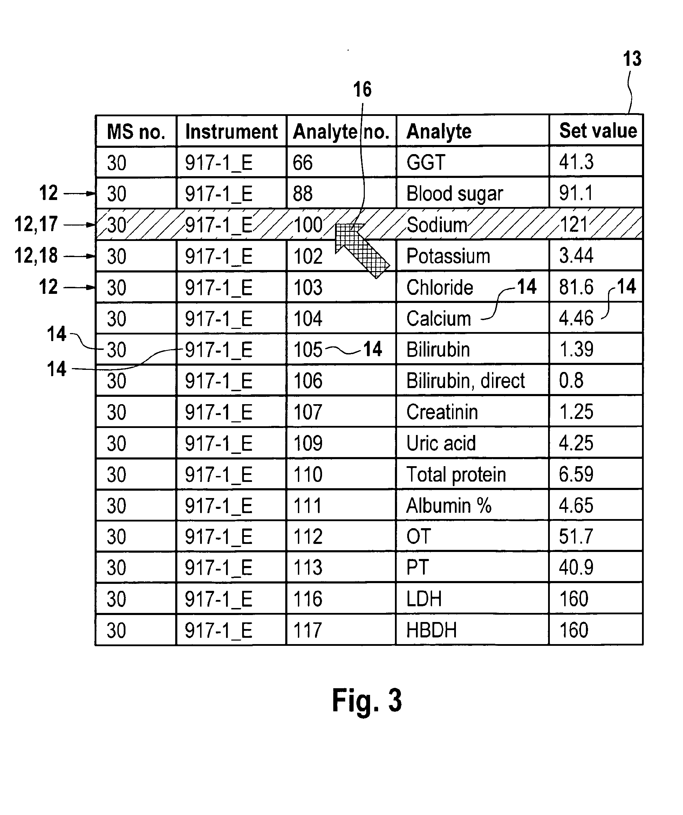

Figure 3 illustrates another example of a selection list 13 comprising similar data records 12 such posing or increasing the risk of wrong selection by the user. It shows a menu used for ordering the processing of a sample, in which menu a list of possible analyses that may be performed for a certain Measurement No. (MS no.) by a laboratory instrument 8 named 917-1_E. Certain analytes which are identified by an analyte number, the name of the analyte and a set value are shown. Here too the possibilities for a user to select a wrong data record 12 are abound.

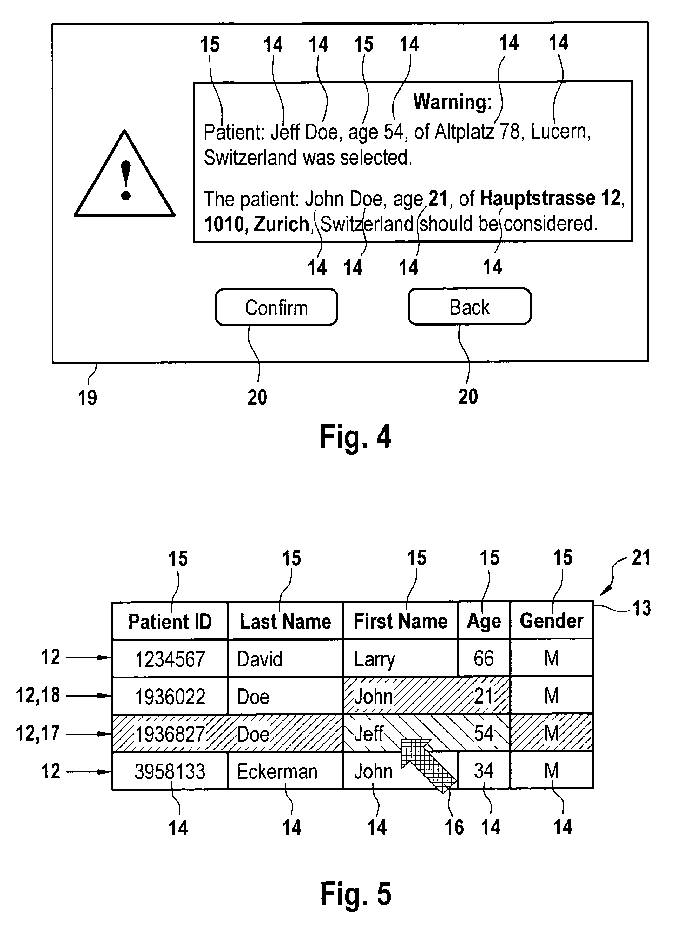

Figures 4 to 7 illustrate several methods according to the invention for reducing data record selection error rates. This may be achieved with respect to a display according to figure 2 or by modifying the display of the selection list 13 of figure 2, e.g. by increasing data record representation distance or by highlighting. These measures according to the invention are generally listed from the simple and easy to the more complex to implement. Of course, two or more of the methods described in this application, also including a downstream verification, can also be combined. Further, the invention is not limited to embodiments according to figure 2 but can in a similar manner also be applied to other embodiments, e.g. according to figure 3.

In the methods illustrated in figures 4 to 7 the data records that are displayed in the displayed selection list 13 are automatically checked for similar data records 18, wherein a similar displayed data record 18 comprises at least one data record attribute 14 of a data type that is identical or similar to the data record attribute 14 of said same data type of another displayed data record 12.

In general, the similarity of data records 12 is checked, e.g. the search for a similar data record 18 among data records 12 displayed in a selection list 13 or among data records 12 displayed with a pointed data record 17 is performed in the following manner: in a table or grid list, attributes are checked for similarity to attribute of same type of other data records. In a linear menu, the various menu entries, each produced from a record's attributes, are checked for similarity to each other. Similarity between text phrases or attributes is evaluated by calculating the text distance or the weighted text distance between them.

According to a preferred embodiment , when a cursor 16 is set by the user to a displayed data record 12 for preparing selection of the data record 12 it is additionally graphically processed.

Figure 4 shows an embodiment wherein the additional graphical processing comprises displaying a data record selection warning 19. Upon data record selection, the system checks if a data record 12, e.g. a data record 17 pointed with a cursor 16 or selected by a name, is among data records 12 with similar representation, and posts a data record selection warning 19 to the user. As shown the display of a data record selection warning 19 may comprise displaying a confirmation prompt 20 to the user to be confirmed by the user for approving and enabling or canceling the selection of data record 12, e.g. the pointed data record 17.

It is to be noted that the data record selection warning 19 is display to the user only if a similar data record 18 similar to the pointed data record 17 exists in the displayed selection list 13. This makes the pattern of data selection irregular and relevant, drawing even more attention to the possibility of error for the user.

Displaying the data record selection warning 19 may comprise displaying at least one similar data records 18 in order to provide the user the required information for making his final selection. In figure 4 this similar record is that of John Doe of the two patients having similar data records 12.

Further, the prompt or data record selection warning 19 may present and highlight the differences between similar "candidates" of similar data records 12, 18 displayed in the selection list 13, thus drawing attention to the differentiating factors, making it easy to identify errors even under time pressure. Accordingly it may be favorable when displaying the data record selection warning 19 comprises displaying at least one different data record attribute 14 of a specific data type which is different for the data record 12, e.g. a pointed data record 17, and the one or more similar data records 18, in particular for the displayed similar data records 18. In figure 4 this different data record attributes 14 are the first name, the age and the different data record entries 14 related to the address information.

Displaying the at least one different data record attribute 14 of the pointed data record 17 and of one ore more similar data records 18 may comprise displaying at least one data record attribute 14 of a specific data type which has been displayed in the displayed selection list 13, here the first name. Displaying the at least one different data record attribute 14 of the pointed data record 17 and of the one ore more similar data record 18 may also in addition or alternatively comprise displaying at least one data record attribute 14 of a specific data type which has not been displayed in the displayed selection list 13, here the differing address information.

According to the preferred embodiment shown at least one displayed different data record attribute 14 is highlighted in the display in order to attract the attention of the user. In figure 4 this applies for the age and the address information. It could also include the first name. The at least one displayed different data record attribute 14 may be highlighted by displaying it with at least one special graphical attribute. The at least one special graphical attribute may be bold font, different font, different font size, italic font, underlined font, different contrast, different color, different contrast color, different background, different background color.

Figure 5 shows an embodiment wherein the additional graphical processing comprises displaying the displayed selection 13 list in a highlighted data record differentiation representation 21, wherein at least one different data record attribute 14 of a specific data type which is different for the data record 12, e.g. a pointed data record 17, and the one or more similar data records 18, in particular the displayed similar data records 18, is highlighted in the pointed data record 12. According to this feature in figure 5 the data record attributes 14 of first name and age of patients Jeff Doe are highlighted.

The additional graphical processing shown in figure 5 also comprises displaying the displayed selection list 13 in a highlighted data record differentiation representation 21, wherein at least one different data record attribute 14 of a specific data type which is different for the data record 12, e.g. a pointed data record 17, and the one or more similar data records 18, in particular the displayed similar data records 18, is highlighted in the one or more similar data records 18. According to this feature in figure 5 the data record attributes 14 of first name and age of patient John Doe are highlighted.

Alternatively or in addition the additional graphical processing may comprise displaying the displayed selection list 13 in a highlighted data record differentiation representation 21, wherein at least one different data record attribute 14 of a specific data type which is different for the pointed data record 17 and the displayed similar data records 18 is highlighted in the displayed similar data records 18. In figure 5 this is the first name and the age of patient John Doe.

The system highlights the differentiating parts of the displayed data records 12 in the selection list 13, i.e. the at least one highlighted data record attribute 14 is highlighted by displaying it with at least one special graphical attribute. The at least one special graphical attribute may be bold font, different font, different font size, italic font, underlined font, different contrast, different color, different contrast color, different background, different background color.

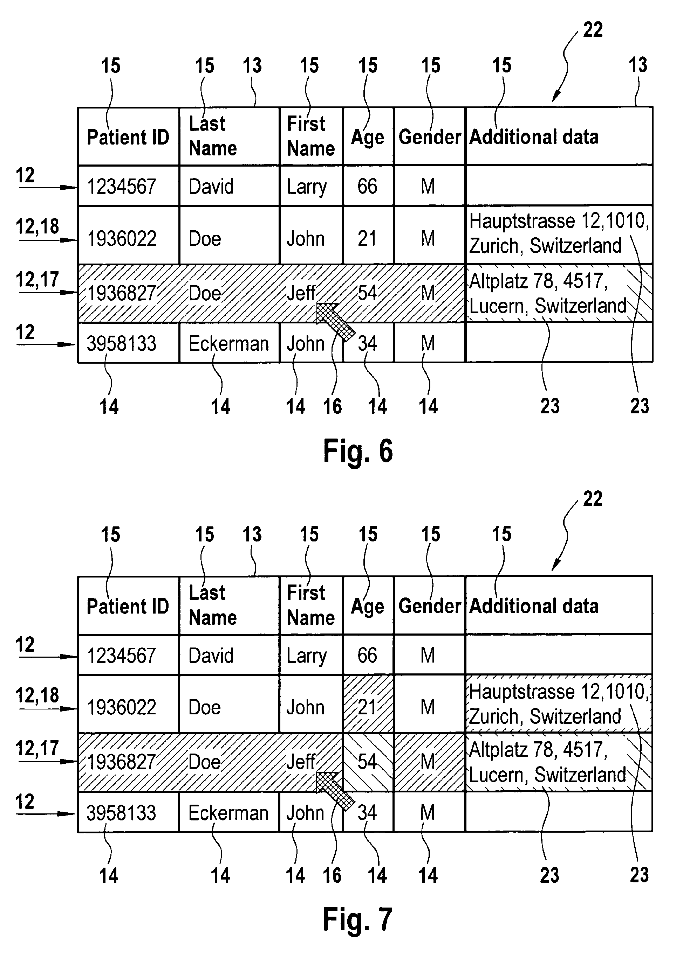

Figures 6 and 7 show an embodiment wherein the additional graphical processing comprises displaying the displayed selection list in a dynamic data record representation 22 displaying an additional data record attribute 23 of at least one additional data type for the data record 12, (e.g. a pointed data record 17), and the one or more similar data records 18 (e.g. the displayed similar data records 18), upon displaying the pointed data record 17 and the one or more similar data records 18. That additional data record attributes 23 displayed are not displayed in the displayed selection list 13 prior to selecting the pointed data record 17 with the cursor 16. In figures 6 and 7 that additional data record entries 23 are data record entries 14 of the data type address. However, data record entries 14 of any other data type can be displayed as additional data record entries 23. The additional differentiating attributes can be displayed statically or dynamically, when the cursor points to one of the similar record representations.

It is to be noted that the additional data record attributes 23 are displayed to the user only if a similar data record 18 similar to the pointed data record 17 exists in the displayed selection list 13. This makes the pattern irregular, drawing even more attention to the possibility of error for the user.

It may be preferable that the data type qualifiers 15 of the additional data record attributes 23 displayed in the selection list 23 are displayed with the displayed additional data record entries 23, e.g. any patient information. In the embodiment shown in figures 6 and 7 a general data type qualifier 15 like "additional data" of the additional data record entries 23 displayed in the selection list 13 is displayed with the displayed additional data record attributes 23.

As can be seen from figures 6 and 7 further the at least one additional data record attribute 23 and/or at least one displayed similar data record attribute 14 can be highlighted in the displayed selection list 13. In figure 6 the additional data record attribute 23 of the address of the pointed data record 17 of patient Jeff Doe is highlighted. In figure 7 also the additional data record attribute 23 of the address of the similar data record 18 of John Doe, the age of John Doe and the age of Jeff Doe are highlighted. Therefore figure 7 provides an even better comparison of the data records 12 than figure 6.

The at least one additional data record attribute 23 and/or at least one displayed similar data record attribute 14 may be highlighted by displaying it with at least one special graphical attribute. The at least one special graphical attribute may be bold font, different font, different font size, italic font, underlined font, different contrast, different color, different contrast color, different background, different background color.

Reference numerals

| 1 | laboratory system |
| 2 | clinical instrument |
| 3 | terminal |
| 4 | database |
| 5 | repository database |
| 6 | user interface |
| 7 | display |
| 8 | laboratory instruments |
| 9 | laboratory information system |
| 10 | communication processor |
| 11 | display processor |
| 12 | data record |
| 13 | selection list |
| 14 | data record attribute |
| 15 | data type qualifier |
| 16 | cursor |
| 17 | pointed data record |
| 18 | similar data record |
| 19 | data record selection warning |
| 20 | confirmation prompt |
| 21 | data record differentiation representation |
| 22 | dynamic data record representation |
| 23 | additional data record attribute |
| 31 | Laboratory information system |
| 32 | Work area manager |
| 33 | Laboratory analyzer data system |
| 34 | Input/Output device |

**Claims**

1. Method for displaying and processing data, wherein

   - data are stored in a repository database,
   - the data are searched or filtered according to a query to the repository database in order to retrieve or preselect a number of data records from the data stored in the repository database.
   - wherein a data record comprises a number of data record attributes, wherein a data record attribute is an instance of a data type stored in the repository database,
   - a selection list comprising at least some of the retrieved data records' representation is displayed to a user for enabling the user to select one of the data records represented in the displayed list,
   - wherein a record representation comprises at least some of the record's attributes or derivations thereof,
   - wherein the displayed data record representations that are displayed in the displayed selection list are automatically checked for similar data record representations, wherein a similar data record representation comprises at least one data record attribute of a data type that is identical or similar to the data record attribute of said data type of another data record, and
   - wherein the display of the similar displayed data record representations is additionally graphically processed upon displaying the selection list.

2. Method according to claim 1, wherein the display of the data record representations and/or of the one or more similar displayed data attributes is additionally graphically processed upon displaying the selection list when a cursor is set by the user to a displayed data record representation.

3. Method according to claim 1, further comprising a data record selection warning when data are selected out of said similar data record representations.

4. Method according to claim 3, wherein displaying the data record selection warning comprises displaying data record representations identical or similar to the selected record representation.

5. Method according to claim 4, wherein displaying the data record selection warning comprises displaying at least one different data record attribute of a specific data type which is different in value for the selected data record and the one or more similar data records.

6. Method according to claim 5, wherein displaying the at least one different data record attribute of the selected data record and of the one ore more similar data record comprises displaying at least one data record attribute of a specific data type which has not been displayed in the displayed selection list.

7. Method according to claim 1, wherein at least one displayed different data record attribute is highlighted in the display.

8. Method according to claim 1, wherein the additional graphical processing comprises displaying the displayed selection list in a highlighted data record differentiation representation, wherein at least one different data record attributes of a specific data type is highlighted.

9. Method according to claim 1, wherein the additional graphical processing comprises displaying the displayed selection list in a dynamic data record representation which displays additional data record attributes of at least one additional data type for the data record representations.

10. Method according to claim 1, wherein upon selecting a data record by the user a confidence level of the selection is recorded together with the selected data record for enabling a downstream verification or correction and a lower confidence level is assigned to the selected data record the more similar data record representations in the displayed selection list have been to the selected data record representation and the higher the number of similar data records has been when the data record was selected.

11. Method according to claim 10, wherein the confidence level is checked if a plausibility check is not passed and the user is prompted an error message when the confidence level assigned is below a threshold.

12. Method according to claim 1, wherein upon selecting a data record by the user the one or more similar data records which are displayed in the selection list are recorded with the selected data record for enabling a downstream verification.

13. A user interface of a laboratory instrument or a laboratory system supporting displaying and processing clinical data to be selected for displaying and processing clinical information, comprising

- a processor for searching or filtering data that are stored in a repository database according to a query to the repository database in order to retrieve a number of data records from the data stored in the repository database,
- wherein a data record comprises a number of data record attributes, wherein a data record attribute is an instance of a data type stored in the repository database, wherein the data type is given by data type qualifiers and
- a display for displaying to a user a selection list comprising at least some of the preselected data record representations for enabling the user to select one or more of the displayed data records,
- wherein the displayed selection list comprises at least some of the data record attributes of the data records which are displayed in the selection list,

wherein a processor

- automatically checks the displayed data record representations that are displayed in the displayed selection list for similar data records, wherein a similar data record comprises at least one data record attribute identical or similar to the data record attribute of said data type of another data record, and
- the display of the data record and/or of a similar data records is additionally graphically processed.

14. Laboratory system comprising an analytical instrument, a terminal for displaying data to a user and a repository database and a user interface according to claim 13.

15. Computer-readable storage device containing a set of instructions that causes a computer to perform a process according to any one of claims 1 to 12.

**To host or other
external systems**

UI,DS

32

31

UI,DS

32'

...

UI,DS

33

UI,DS

33'

UI,DS

33"

...

...

UI,DS

34

34'

34"

UI,DS - User Interaction, Data Selection

## Fig. 0

Fig. 1

| Patient ID | Last Name | First Name | Age | Gender |
|---|---|---|---|---|
| 1234567 | David | Larry | 66 | M |
| 1936022 | Doe | John | 21 | M |
| 1936827 | Doe | Jeff | 54 | M |
| 3958133 | Eckerman | John | 34 | M |

**Fig. 2a**

Larry David
John Doe
Jeff Doe
John Eckerman

Add another form of selection list

**Fig. 2b**

| MS no. | Instrument | Analyte no. | Analyte | Set value |
|---|---|---|---|---|
| 30 | 917-1_E | 66 | GGT | 41.3 |
| 30 | 917-1_E | 88 | Blood sugar | 91.1 |
| 30 | 917-1_E | 100 | Sodium | 121 |
| 30 | 917-1_E | 102 | Potassium | 3.44 |
| 30 | 917-1_E | 103 | Chloride | 81.6 |
| 30 | 917-1_E | 104 | Calcium | 4.46 |
| 30 | 917-1_E | 105 | Bilirubin | 1.39 |
| 30 | 917-1_E | 106 | Bilirubin, direct | 0.8 |
| 30 | 917-1_E | 107 | Creatinin | 1.25 |
| 30 | 917-1_E | 109 | Uric acid | 4.25 |
| 30 | 917-1_E | 110 | Total protein | 6.59 |
| 30 | 917-1_E | 111 | Albumin % | 4.65 |
| 30 | 917-1_E | 112 | OT | 51.7 |
| 30 | 917-1_E | 113 | PT | 40.9 |
| 30 | 917-1_E | 116 | LDH | 160 |
| 30 | 917-1_E | 117 | HBDH | 160 |

## Fig. 3

**Warning:**

Patient: Jeff Doe, age 54, of Altplatz 78, Lucern, Switzerland was selected.

The patient: John Doe, age 21, of **Hauptstrasse 12, 1010, Zurich,** Switzerland should be considered.

| Confirm | Back |

## Fig. 4

| Patient ID | Last Name | First Name | Age | Gender |
|---|---|---|---|---|
| 1234567 | David | Larry | 66 | M |
| 1936022 | Doe | John | 21 | M |
| 1936827 | Doe | Jeff | 54 | M |
| 3958133 | Eckerman | John | 34 | M |

## Fig. 5

| Patient ID | Last Name | First Name | Age | Gender | Additional data |
|---|---|---|---|---|---|
| 1234567 | David | Larry | 66 | M | |
| 1936022 | Doe | John | 21 | M | Hauptstrasse 12,1010, Zurich, Switzerland |
| 1936827 | Doe | Jeff | 54 | M | Altplatz 78, 4517, Lucern, Switzerland |
| 3958133 | Eckerman | John | | 34 | M | |

**Fig. 6**

| Patient ID | Last Name | First Name | Age | Gender | Additional data |
|---|---|---|---|---|---|
| 1234567 | David | Larry | 66 | M | |
| 1936022 | Doe | John | 21 | M | Hauptstrasse 12,1010, Zurich, Switzerland |
| 1936827 | Doe | Jeff | 54 | M | Altplatz 78, 4517, Lucern, Switzerland |
| 3958133 | Eckerman | John | | 34 | M | |

**Fig. 7**

Europäisches
Patentamt

European
Patent Office

Office européen
des brevets

## EUROPEAN SEARCH REPORT

Application Number

EP 08 10 5816

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/093312 A (HILL ROM SERVICES INC [US]; BAGNELL JOSEPH P [US]; BOONE OTHO N [US];) 21 November 2002 (2002-11-21) * abstract * * page 2, line 10 - page 5, line 5 * * page 8, line 12 - page 52, column 16; claims 1-49; figures 1-25 * ----- | 1-15 | INV. G06F19/00 G06F3/048 |
| X | WO 2008/134098 A (MEDTRONIC INC [US]; WENGER WILLIAM K [US]; GARG CHAYA K [US]; HOEPPNER) 6 November 2008 (2008-11-06) * abstract * * page 2, line 1 - page 5, line 4 * * page 6, line 1 - page 27, line 29; claims 1-8; figures 1-9 * ----- | 1-15 | |
| X | WO 02/39250 A (SIEMENS MEDICAL SOLUTIONS [US]) 16 May 2002 (2002-05-16) * abstract * * page 2, line 21 - page 4, line 21 * * page 6, line 11 - page 26, line 18; claims 1-20; figures 1-9 * ----- | 1-15 | |
| A | US 2007/027890 A1 (POYOUROW DAVID [US] ET AL) 1 February 2007 (2007-02-01) * the whole document * ----- | 1-15 | TECHNICAL FIELDS SEARCHED (IPC)<br><br>G06F |

The present search report has been drawn up for all claims

| Place of search | Date of completion of the search | Examiner |
|---|---|---|
| The Hague | 9 March 2009 | Wierzejewski, Piotr |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 08 10 5816

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2009

| Patent document cited in search report | | Publication date | Patent family member(s) | | Publication date |
|---|---|---|---|---|---|
| WO 02093312 | A | 21-11-2002 | AU | 2002309838 A1 | 25-11-2002 |
| | | | CA | 2445520 A1 | 21-11-2002 |
| | | | EP | 1388106 A2 | 11-02-2004 |
| | | | JP | 2005514078 T | 19-05-2005 |
| WO 2008134098 | A | 06-11-2008 | US | 2008270188 A1 | 30-10-2008 |
| WO 0239250 | A | 16-05-2002 | CN | 1608270 A | 20-04-2005 |
| | | | EP | 1374029 A2 | 02-01-2004 |
| | | | JP | 2004513452 T | 30-04-2004 |
| | | | US | 2002065686 A1 | 30-05-2002 |
| US 2007027890 | A1 | 01-02-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6775670 B

- US 7165221 B